# FASCICULE DE BREVET EUROPEEN

(11) **EP 1 863 821 B1**
(45) Date de publication et mention de la délivrance du brevet: **01.08.2012**
(21) Numéro de dépôt: 06726130.5
(22) Date de dépôt: 22.03.2006
(51) Int. Cl.: C07D 493/04

(54) **Procédé de préparation de compositions de diester(s) de dianhydrohexitol**
Verfahren zur Herstellung von Dianhydrohexitoldiesterzusammensetzungen
Method for preparing dianhydrohexitol diester compositions

(30) Priorité: 01.04.2005 FR 0503241
(43) Date de publication de la demande: 12.12.2007
(73) Titulaire: ROQUETTE FRERES, 62136 Lestrem (FR)
(72) Inventeur: FUERTES, Patrick, F-59160 Lomme (FR); WYART, Hervé, F-62149 Cuinchy (FR)
(74) Mandataire: Cabinet Plasseraud
(86) Numéro de dépôt international: PCT/FR2006/000632
(87) Numéro de publication internationale: WO 2006/103338

(56) Documents cités:
- EP-A- 0 065 267
- WO-A-01/83488
- WO-A-99/45060
- US-A- 2 322 821
- US-A- 4 297 290
- DATABASE CA [Online] CHEMICAL ABSTRACTS SERVICE, COLUMBUS, OHIO, US; YASUDA, MINORU ET AL YASUDA, MINORU ET AL: "Sorbitol esters with higher fatty acids Sorbitol esters with higher fatty acids" XP002357477 extrait de STN Database accession no. 1969:79433 & JP 44 002964 B (JAPAN OILS AND FATS CO., LTD.) 7 février 1969 (1969-02-07) cité dans la demande

## Description

La présente invention a pour objet un nouveau procédé de préparation de compositions de diester(s)de dianhydrohexitols tels que l'isosorbide, l'isoidide, l'isommanide ou l'isogalactide.

Elle concerne également, en tant que produits industriels nouveaux, certaines de ces compositions, sélectionnées notamment pour leurs caractéristiques de richesse en diester(s) et/ou de coloration.

Elle vise enfin l'utilisation des compositions précitées dans différents domaines industriels, en particulier dans les matières plastiques.

La préparation d'esters d'anhydrohexitols est connue depuis plus de 60 ans et a notamment été décrite dans les brevets ci-dessous mentionnés, publiés dans les années 40 au nom de la Société Atlas Powder Company :
- US 2,322,820 relatif à la préparation de compositions de monoesters d'hexitans (sorbitan, mannitan) et/ou d'hexides (sorbide = isosorbide ou mannide = isomannide),
- US 2,322,821 relatif à la préparation de compositions de monoesters d'isosorbide ou d'isommanide, lesdites compositions pouvant contenir des taux significatifs de diesters d'hexides,
- US 2,387,842 relatif à la préparation de diesters ou diesters mixtes d'isosorbide ou d'isommanide.

Dans ce dernier brevet, la préparation de diesters est exemplifiée soit à partir d'un hexitol (sorbitol, mannitol) soit d'un dianhydrohexitol (isosorbide ou isommanide). La possibilité de partir d'un monoanhydrohexitol (ou hexitan) est également évoquée sans être exemplifiée.

En tout état de cause, que l'on parte d'hexitol ou de dianhydrohexitol, la réaction proprement dite d'estérification se fait systématiquement en la présence de toluène en tant que moyen d'élimination d'eau et, dans la quasi-totalité des exemples, en présence d'un catalyseur acide, ce dernier étant toujours de l'acide sulfurique concentré.

Après neutralisation et rinçage à l'eau, le milieu réactionnel subit uniquement un traitement d'évaporation du toluène sous vide.

Les esters obtenus sont alors testés comme plastifiants de matières plastiques (chlorure, chloro-acétate et butyrol de polyvinyle).

A la même époque, le brevet GB 613,444 évoque la préparation, à partir d'isosorbide, de di-tétrahydro-furcate ou di-butyrate d'isosorbide avec élimination d'eau en continu par reflux puis simple distillation sous vide du milieu réactionnel.

Les esters d'isosorbide sont présentés comme de bons agents de ramollissement ou plastifiants de résines et de matériaux cellulosiques.

En 1953, Y. HACHIHAMA et I. HAYASHI (Techno. Repts. Osaka Univ. 1953, Vol. 3, pp. 191-200) confirment l'intérêt des diesters d'isosorbide comme plastifiants de polychlorure de vinyle (PVC). L'estérification se fait en présence d'acide sulfurique ou d'acide para-toluène sulfonique (APTS) et, dans la quasi totalité des exemples, en présence de toluène ou de xylène. Il n'est pas fait mention d'un quelconque moyen additionnel de traitement, en particulier de purification.

Le brevet US 3,023,223 décrit la préparation d'isoidide (1,4-3,6-dianhydro-L-iditol) en précisant simplement qu'il peut être converti en diesters utilisables comme plastifiants de résines synthétiques par estérification avec des acides mono-carboxyliques.

Le brevet US 3,454,603 décrit la préparation d'isoidide et d'isosorbide en précisant que par estérification avec des acides ou esters gras, ils peuvent être transformés en agents tensio-actifs.

A titre illustratif il est fait uniquement référence à la préparation potentielle de monostéarates d'isoidide ou isosorbide en présence de 0,05 % d'APTS à 200°C pendant 4 heures sous atmosphère inerte.

Le brevet JP 44-2964 décrit la préparation d'agents tensioactifs à base d'esters de monoanhydrohexitols, en l'occurrence d'esters de sorbitan de type « SPAN ». Les auteurs précisent que pour obtenir une moindre coloration finale desdits esters, il convient de mettre en oeuvre un système catalytique associant obligatoirement et dans des proportions très précises, un agent alcalin (par exemple du carbonate de sodium) et, soit de l'acide phosphorique, soit de l'acide hypophosphoreux ou leurs sels. Il apparaît que l'acide phosphorique permet, dans ces conditions, d'atteindre une coloration moins prononcée que celle obtenue avec l'acide hypophosphoreux. Cependant, cette coloration ne semble pas acceptable et impose la mise en oeuvre d'un traitement supplémentaire de décoloration par du chlorite de sodium ou de l'eau oxygénée.

Le brevet EP 65.267 confirme l'usage de catalyseurs alcalins dans la préparation d'esters de monoanhydrohexitols et la nécessité d'opérer un traitement de blanchiment par eau oxygénée (H₂O₂) en vue d'obtenir des produits présentant une coloration correcte.

A la même époque, le brevet US 4,297,290 publié en 1981 décrit la fabrication d'esters de sorbitan selon laquelle la réaction d'estérification se fait en présence d'une base et à une température n'excédant par 215°C en vue d'obtenir des produits de coloration améliorée.

Selon les exemples de ce brevet, l'estérification se fait cependant en présence de charbon actif comme moyen de décoloration.

En outre, le milieu réactionnel est a) neutralisé par de l'acide phosphorique en vue d'en limiter la coloration lors d'un test de stabilité à 93°C puis b) traité par de l'eau oxygénée et, à chaque fois, filtré en présence de terre de diatomées.

Beaucoup plus récemment, la demande de brevet WO 99/45060 exemplifie, sans la détailler véritablement, la préparation de diesters particuliers d'isosorbide ou d'isomannide en vue de leurs utilisations comme solvants ou plastifiants de polymères.

Cette préparation se fait à partir de dianhydrohexitols, en présence de 4 % d'APTS et d'un solvant (xylène). Le brut réactionnel refroidi est traité par un autre solvant (diéthyléther) puis rincé à l'eau (sodée) et évaporé. Selon les exemples, les rendements en diesters vont de 86 à 95 %. Cependant, aucun détail n'est donné ou ne peut être déduit quant aux conditions exactes, notamment de température, dans lesquelles le milieu réactionnel est chauffé/mis en ébullition et, surtout, évaporé, ces conditions jouant obligatoirement sur la coloration du produit final.

En dernier lieu, la demande de brevet WO 01/83488 décrit un procédé amélioré de préparation d'esters de sorbitan ou d'isosorbide par mise en oeuvre d'un catalyseur acide de type résine échangeuse d'ions acide macroporeuse. Selon les auteurs, cette mise en oeuvre doit permettre d'obtenir avec de hauts taux de conversion (98 % - 100 %) des produits présentant une coloration substantiellement améliorée, y compris en regard des produits divulgués dans la demande WO 99/45060 précitée et donc permettre de s'affranchir de toute étape de distillation.

Cette amélioration de coloration est présentée comme résultant de la possibilité d'effectuer, grâce aux dites résines macroporeuses, une réaction d'estérification à une température inférieure à 150°C.

Par ailleurs, les auteurs soulignent la possibilité d'obtenir les mêmes effets en partant, non pas d'isosorbide, mais de sorbitol ou de sorbitan dès lors que la réaction de déshydratation se fait à température relativement basse (120-125°C) avant d'augmenter cette température à 140-150°C en vue de l'estérification proprement dite.

En tout état de cause, ce procédé présente le désavantage d'être coûteux du fait même de l'utilisation desdites résines macroporeuses. En effet, outre leur prix élevé, ces catalyseurs sont mis en oeuvre dans des proportions importantes, à savoir de l'ordre de 13 % (en poids sec/poids sec d'isosorbide) selon les exemples de ce brevet.

La majorité desdits exemples montre par ailleurs :
1. la nécessité toujours présente de distiller le milieu réactionnel en vue d'éliminer l'acide gras (acides n-octanoïque ou 2-éthylhexanoïque) en excès,
2. la nécessité d'éliminer la résine macroporeuse et ce, par filtration du milieu réactionnel préalablement refroidi à 60-80°C,
3. la nécessité de traiter ensuite ledit milieu réactionnel avec du charbon actif après avoir cependant réchauffé ledit milieu à une température de 80-100°C.

Indépendamment de la quantité exacte de charbon actif mise en oeuvre après élimination de la résine macroporeuse, ladite quantité n'étant pas précisée dans cette demande WO 01/83488, on peut admettre que le procédé ainsi envisagé est complexe en pratique puisqu'il impose deux étapes de filtration, l'une pour éliminer la résine macroporeuse, la seconde pour éliminer le charbon actif.

Il n'en reste pas moins que ce procédé ne permet pas d'obtenir un milieu réactionnel traité sur charbon actif, évaporé ou non, qui soit véritablement incolore.

Au mieux, la couleur de ce milieu est qualifiée de « jaune pâle » (« pale yellow »), sans d'ailleurs que ne soit indiquée la moindre valeur de coloration en fonction d'une quelconque technique de mesure qui aurait été elle-même décrite.

L'EXEMPLE 5 de ce brevet décrit l'obtention finale d'une composition de 2,5-di-n-octanoate d'isosorbide, apparemment moins colorée, puisque qualifiée de « virtuellement claire comme de l'eau » (« virtually « water-white » ») sans qu'aucune mesure de coloration n'ait été effectuée.

En tout état de cause, le procédé décrit spécifiquement dans cet EXEMPLE 5 est compliqué et coûteux puisqu'il envisage:
a) la mise en oeuvre de charbon actif en deux endroits et en des quantités importantes, à savoir 1) à raison de 7 % en poids de matière seche/matière sèche d'isosorbide pendant la réaction d'estérification puis encore 2) à raison de 3,5 % en poids de matière sèche/matière sèche d'isosorbide après distillation,
b) un double traitement, après distillation du milieu réactionnel résultant, par 1) un solvant organique, en l'occurrence du n-hexane, puis 2) par du charbon actif (cf. supra).

Ce procédé est d'autant plus compliqué et coûteux que la résine macroporeuse mise en oeuvre de manière concomitante au charbon actif lors de la réaction d'estérification ne peut pas être réutilisée efficacement puisqu'elle est polluée par ledit charbon et par les espèces colorantes absorbées par celui-ci.

En ce qui concerne spécifiquement la préparation de diesters de dianhydrohexitols, il apparaît que malgré les moyens à la portée de l'homme de l'art, susceptibles d'aider à la purification et/ou la décoloration de ces produits et plus largement des esters d'anhydrohexitols, il n'a pas été possible jusqu'à ce jour de disposer d'un procédé industriel qui soit à la fois simple, économique, performant et sans danger et en particulier un procédé qui, simultanément :
a) n'impose pas la mise en oeuvre obligatoire de résines macroporeuses lors de la réaction d'estérification mais soit applicable également à des catalyseurs classiques moins onéreux tels que, par exemple, l'acide sulfurique ou l'APTS,
b) permette d'obtenir des degrés élevés de richesse en diesters de dianhydohexitols, à savoir des richesses supérieures à 90 %, de préférence au moins égales à 95 % et plus préférentiellement encore au moins égales à 98% et ce, sans obligatoirement mettre en oeuvre un moyen de purification autre qu'un traitement classique de distillation et en particulier sans utilisation du moindre solvant organique,
c) n'impose pas la mise en oeuvre obligatoire de plusieurs étapes de traitement par charbon actif et/ou de quantités importantes (à savoir supérieures à 3-4 %, en particulier au moins égales à 5 %, exprimées en poids de matière sèche par rapport au poids sec de dianhydrohexitol de départ) de charbon actif en vue d'obtenir des produits de coloration acceptable,
d) n'impose pas la mise en oeuvre obligatoire d'eau oxygénée en vue d'obtenir des produits de coloration acceptable,
e) permette d'améliorer encore la coloration de compositions intermédiaires ou finales de tels diesters et ce, sans nuire à l'économie générale du procédé,
f) soit avantageusement applicable non seulement à la préparation de diesters d'isosorbide ou d'isommanide, préparation relativement documentée dans l'art antérieur, mais encore à celle de diesters d'autres dianhydrohexitols et en particulier à la préparation, jamais véritablement exemplifiée jusqu'alors, de diesters d'isoidide.

Et le mérite de la Demanderesse est d'avoir trouvé, après de nombreux travaux de recherche et d'analyse, qu'un tel moyen consistait en un procédé présentant la double caractéristique:
1) de partir obligatoirement de dianhydrohexitol, et non pas d'hexitol ou d'hexitan (monoanhydrohexitol), en tant que matière première et,
2) de mettre en oeuvre obligatoirement, lors de l'estérification, a) un catalyseur acide et b) de l'acide hypophosphoreux.

La Demanderesse a tout d'abord observé lors d'une première série d'études, qu'il n'était pas possible, en partant d'hexitol (par exemple de sorbitol), de préparer une composition présentant une richesse en diester de dianhydrohexitol (par exemple en di-n-octanoate d'isosorbide) dépassant ou même atteignant la valeur de 85 %. Parmi les très nombreux catalyseurs acides testés, les résines macroporeuses se sont révélées, à cette occasion, être les plus efficaces sans toutefois permettre d'obtenir une richesse en diesters supérieure à 78-81 % environ. Un catalyseur classique tel que l'APTS n'a pas permis ici d'atteindre une richesse en diester de 70 % et ce, même en faisant varier les conditions opératoires (ratio APTS/sorbitol, conditions de mise en oeuvre de l'acide n-octanoïque, températures de réaction...). En effet, en partant d'hexitol comme matière première, il a été observé dans tous les cas, y compris en présence de résine macroporeuse comme catalyseur, une co-production très importante d'espèces autres que le(s) diester(s). recherché(s), en particulier de tri- et tétraesters de monoanhydrohexitol (par exemple de tri- et tétraoctanoates de sorbitan).

C'est pourquoi, lors d'une seconde série d'études, la Demanderesse a pris résolument le parti d'utiliser un dianhydrohexitol (par exemple l'isosorbide) en tant que matière première en vue de préparer tout diester recherché (par exemple le di-octanoate d'isosorbide).

A cette occasion, il a été constaté que non seulement il était possible d'obtenir des compositions riches en diester(s) sans obligatoirement mettre en oeuvre une résine macroporeuse mais en utilisant tous types de catalyseurs acides d'estérification, mais encore que la mise en oeuvre supplémentaire d'acide hypophosphoreux, lors de la réaction d'estérification, permettait de procurer des effets spécifiques et particulièrement avantageux en termes d'absence de coloration de la composition de diester(s), non seulement à l'état de brut réactionnel mais surtout après que le diester ait subi un traitement d'évaporation en vue de l'élimination par distillation de l'acide carboxylique en excès, éventuellement suivi d'un traitement classique de décoloration par charbon actif et/ou eau oxygénée.

La Demanderesse a notamment constaté que, de façon surprenante et inattendue, la mise en oeuvre d'acide hypophosphoreux (H₃PO₂) lors de la réaction d'estérification permettait :
- d'obtenir des bruts réactionnels beaucoup moins colorés qu'avec la mise en oeuvre d'acides très proches structurellement tels que l'acide phosphorique (H₃PO₄), plus particulièrement recommandé dans le brevet JP 44-2964 pour les esters de sorbitan, ou l'acide orthophosphoreux (H₃PO₃),
- d'obtenir, après purification par évaporation desdits bruts réactionnels, des compositions dont la coloration était identique voire inférieure à la coloration avant purification par évaporation, alors qu'en absence d'acide hypophosphoreux ou en présence de charbon actif lors de l'étape antérieure d'estérification, les compositions obtenues après purification par évaporation présentaient une coloration significativement plus importante,
- d'obtenir, après purification par évaporation et traitement classique de décoloration, des compositions de diester(s) de dianhydrohexitol de richesse élevée, exemptes de traces de solvants organiques et présentant une absence ou quasi-absence de coloration jamais atteinte jusqu'alors.

En suite de quoi, la présente invention a pour objet un procédé de préparation d'une composition de diester(s) de dianhydrohexitol, caractérisé en ce qu'il comprend une étape au cours de laquelle on soumet une composition de dianhydrohexitol à une estérification par un acide carboxylique en présence d'un catalyseur acide et d'acide hypophosphoreux.

La composition de dianhydrohexitol utilisée comme matière première peut notamment être une composition d'isosorbide, d'isommanide, d'isoidide ou d'isogalactide.

Il peut également s'agir d'un mélange de ces dianhydrohexitols.

Cette composition de dianhydrohexitol peut avoir été préalablement obtenue par toute technique connue de déshydratation d'un hexitol ou d'un mélange d'hexitols, généralement suivie, après neutralisation, d'au moins une technique de purification du brut réactionnel ainsi obtenu.

L'étape de purification peut consister en une simple distillation du milieu issu de la déshydratation. En suite de quoi, la composition de dianhydrohexitol consiste en un distillat brut, par exemple en un distillat brut d'isosorbide, d'isommanide et/ou d'isoidide.

Ledit distillat peut cependant avoir subi au moins une étape supplémentaire de purification, et notamment :
- par cristallisation en phase aqueuse ou en phase solvant,
- par concentration sous vide, et/ou,
- par traitement sur résine(s) échangeuse(s) d'ions et charbon actif, sous forme poudre et/ou granulaire, comme décrit dans la demande WO 01/94352 au nom de la Demanderesse.

En tout état de cause, qu'il y ait ou non purification après distillation, la composition de dianhydrohexitol mise en oeuvre comme matière première dans le procédé selon l'invention, présente avantageusement une richesse en dianhydrohexitol(s) au moins égale à 95 %, de préférence au moins égale à 98 % et plus préférentiellement encore au moins égale à 98,5 %, ces pourcentages étant exprimés en poids sec total de dianhydrohexitol(s) par rapport au poids sec de ladite composition.

L'acide carboxylique mis en oeuvre en vue de l'estérification peut notamment être tout acide ou tout mélange d'acides décrit dans l'un quelconque des documents précités, en particulier dans les documents US 2,387,842, EP 65267, WO 99/45060 ou WO 01/83488 précités.

L'acide carboxylique mis en oeuvre, seul ou en mélange, peut avantageusement être un acide en C₂ à C₂₄-Il peut s'agir, par exemple, d'acide acétique, d'acide n-octanoïque, d'acide 2-éthylhexanoïque ou d'un mélange contenant l'un au moins de ces acides.

Le catalyseur acide mis en oeuvre en vue de l'estérification peut, comme déjà souligné, être de nature très variée et consister non seulement en une résine macroporeuse, mais également en un autre catalyseur acide choisi, en particulier, dans le groupe formé par les résines non macroporeuses, l'acide chlorhydrique, l'acide sulfurique, l'acide para-toluène sulfonique (APTS), l'acide méthanesulfonique, l'acide trifluorométhanesulfonique, l'acide trifluoroacétique, l'acide trichloroacétique, l'éthyl-2-hexanoate d'étain, l'acide phosphotungstique et l'acide silicotungstique. Il peut s'agir d'un mélange d'au moins deux des catalyseurs acides précités.

La Société Demanderesse a observé que ledit catalyseur acide pouvait avantageusement consister en APTS, en acide méthanesulfonique, ou en acide phosphotungstique.

Lorsque le catalyseur acide est une résine, macroporeuse ou non, il peut être introduit en quantité inférieure à la quantité décrite dans les exemples de la demande de brevet WO 01/83488 précitée, i.e. en une quantité inférieure à 13,7 % environ, exprimée en poids sec par rapport au poids sec de dianhydrohexitol(s) mis en oeuvre. Cette quantité peut notamment être au plus égale à 12 % et notamment comprise entre 5 et 10 %.

Lorsque le catalyseur acide n'est pas une résine, macroporeuse ou non, il peut avantageusement être introduit en une quantité comprise entre 0,05 et 5 %, de préférence comprise entre 0,2 et 4 % de poids sec rapporté au poids sec de dianhydrohexitol(s).

Cette quantité peut notamment se situer entre 0,3 et 3 %, y compris lorsque ledit catalyseur est l'APTS, l'acide méthanesulfonique ou l'acide phosphotungstique.

Les conditions générales de l'estérification (notamment le rapport molaire dianhydrohexitol(s)/acide(s) carboxylique(s), la température réactionnelle et la durée réactionnelle, les moyens d'élimination de l'eau) sont celles classiquement mises en oeuvre dans la littérature en vue de la préparation recherchée de compositions à richesse élevée en diester(s) de dianhydrohexitol.

Comme souligné précédemment, une caractéristique essentielle de la présente invention est de prévoir, lors de la réaction d'estérification, la présence d'acide hypophosphoreux (H₃PO₂) .

Celui-ci peut être introduit dans le milieu réactionnel simultanément ou non au catalyseur acide et/ou à l'acide carboxylique

Selon une variante, cette introduction est opérée avant le début de la réaction d'estérification, i. e. avant l'introduction du catalyseur acide et/ou de l'acide carboxylique.

Selon une variante préférentielle, cette introduction est opérée dès le début de la réaction d'estérification.

De manière avantageuse et quel que soit le moment de son introduction, l'acide hypophosphoreux est introduit en une quantité comprise entre 0,05 et 2 %, de préférence comprise entre 0,1 et 1 %, exprimée en poids sec par rapport au poids sec de dianhydrohexitol(s) mis en oeuvre.

Selon une autre variante préférentielle, l'acide hypophosphoreux est introduit, simultanément ou non au catalyseur acide d'estérification, selon un rapport acide hypophosphoreux/catalyseur acide inférieur à 1/1, ledit rapport étant exprimé en poids sec d'acide hypophosphoreux rapporté au poids sec de catalyseur acide.

Ledit rapport peut notamment être compris entre 0,01/1 et 0,9/1, de préférence compris entre 0,02/1 et 0,8/1.

Lorsque le catalyseur est l'ATPS, l'acide méthanesulfonique ou l'acide phosphotungstique, ledit ratio peut être avantageusement compris entre 0,05/1 et 0,4/1.

Après estérification, le brut réactionnel est de préférence soumis, directement ou non, à au moins une étape de purification. Celle-ci consiste avantageusement en au moins une étape d'évaporation permettant d'éliminer, par distillation, la plus grande partie ou la quasi-totalité, voir la totalité, de l'acide carboxylique éventuellement encore présent dans ce brut réactionnel.

Lors de cette étape, la composition de diester(s) de dianhydrohexitol est soumise, au sein du réacteur ou de l'évaporateur, à des conditions de températures élevées, comprises entre 100 °C et 250 °C, et de pression réduite, comprises entre 0,001 mbars et 50 mbars, conditions qui colorent fortement des compositions de diesters de dianhydrohexitols préparées en l'absence d'acide hypophosphoreux.

De préférence, cette étape se fait dans un évaporateur continu.

Un tel dispositif, par exemple de type « à flot tombant » ou mieux, de type « à film raclé » ou « short path », permet de limiter les températures et temps de séjour auxquels sont ainsi soumis les bruts réactionnels.

En suite de quoi, la présente invention concerne un procédé tel que décrit précédemment et caractérisé, en outre, en ce qu'il comprend une étape subséquente d'évaporation du milieu issu, directement ou non, de l'étape d'estérification, ladite étape étant, de préférence, menée dans un évaporateur continu.

On dispose ainsi d'un moyen permettant de préparer efficacement une composition de diester(s) de dianhydrohexitol(s) présentant une richesse en diester(s) élevée, à savoir au moins égale à 95 %, de préférence au moins égale à 98 %, cette richesse étant exprimée en poids sec total de diester(s) de dianhydrohexitol(s) par rapport au poids sec de ladite composition.

Cette richesse, par exemple en di n-octanoate d'isosorbide (seul diester obtenu), en di n-octanoate d'isoidide (seul diester obtenu) ou en di n-octanoate d'isosorbide + di n-octanoate d'isoidide (obtenus en mélange), est avantageusement au moins égale à 98,5 %.

Elle peut être évaluée par toute méthode mise à la disposition de l'homme de l'art et notamment par chromatographie en phase gazeuse, par exemple sur colonne capillaire diméthylpolysiloxane (DB1) avec utilisation d'un Détecteur à Ionisation de Flamme (« FID »). L'échantillon est injecté sous la forme de dérivé pertriméthylsilylé (bis(triméthylsilyl)trifluoroacétamide (BSTFA)/triméthylchlorosilane (TMCS)/pyridine). Les conditions chromatographiques doivent permettre de séparer les composés de l'acide carboxylique, par exemple de l'acide octanoïque ou de l'acide éthylhexanoïque, éventuellement encore présent ainsi que des tétraesters de monoanhydrohexitols éventuellement présents.

Les diesters de dianhydrohexitols sont repérés par leurs temps de rétention relatifs par rapport au méthyl-alpha-0-glucopyranoside.

La quantification de ces diesters se fait par la méthode de normalisation interne.

Le procédé objet de l'invention permet d'obtenir efficacement des compositions présentant à la fois des richesses élevées en diester(s) et des indices de coloration très faibles qui, pour certains; n'ont encore jamais été atteints jusqu'alors.

La mise en oeuvre, conformément à l'invention, d'acide hypophosphoreux lors de l'étape d'estérification permet notamment d'obtenir des compositions de diester(s) de dianhydrohexitol présentant des valeurs d'indice de jaune YI (« Yellow Index ») considérablement plus faibles que celles obtenues en l'absence de cet acide particulier.

La valeur d'indice de jaune YI est ici mesurée conformément à la norme ASTM D 1925-70, en particulier en utilisant le colorimètre ColorFlex^{™} et son guide d'utilisation tels que fournis par Hunterlab.

La « source » ou « illuminant » consiste en la « CIE Source C » ou « CIE Illuminant C ».

L'observateur consiste en le « 1931 CIE 2 standard observer ».

La Société Demanderesse a constaté que le procédé selon l'invention permettait d'obtenir, après évaporation et avant toute étape complémentaire éventuelle de purification et/ou de décoloration, une composition de diester(s) présentant un indice de jaune YI au plus égal à 50.

De manière tout à fait remarquable, cet indice peut même être au plus égal à 45, voire au plus égal à 40.

Il convient de rappeler, comme déjà souligné, que l'étape d'évaporation a pour effet certes d'augmenter (très) significativement la richesse en diester(s) de dianhydrohexitol de la composition par rapport au brut réactionnel de départ mais a généralement également pour effet concomitant indésirable, comme l'a vérifié en de très nombreuses occasions la Demanderesse, d'augmenter très significativement la coloration du produit résultant.

Ceci, en particulier, quand l'étape d'estérification se fait soit en absence d'acide hypophosphoreux, soit en la seule présence de charbon actif comme décrit dans l'EXEMPLE 5 de la demande de brevet WO 01/83488 précitée.

Il a été constaté, de façon particulièrement surprenante, que la mise en oeuvre d'acide hypophosphoreux lors de l'étape d'estérification permettait d'obtenir, directement après évaporation, des compositions de diesters présentant une coloration identique voire plus faible que celle des bruts réactionnels non soumis à évaporation. Cette coloration peut notamment être caractérisée, comme indiqué supra, par un indice de jaune YI au plus égal à 50, valeur toujours dépassée pour une composition après évaporation obtenue à partir d'une étape d'estérification menée en absence d'acide hypophosphoreux et éventuellement en présence de charbon actif.

La Société Demanderesse considère d'ailleurs comme un produit nouveau et inventif une composition de diester(s) de dianhydrohexitol, obtenue par le procédé selon l'invention, caractérisée en ce qu'elle consiste en un produit résultant de l'estérification d'un dianhydrohexitol et d'un acide carboxylique, puis d'une évaporation, et en ce qu'elle présente une richesse en diester(s) au moins égale à 95 % et un indice de jaune YI au plus égal à 50, de préférence au plus égal à 45.

Avantageusement, cette richesse en diester(s) de dianhydrohexitol peut être au moins égale à 98 % et/ou cet indice YI peut être au plus égal à 40, notamment au plus égal à 35.

Ces valeurs sont d'autant plus remarquables qu'elles ne peuvent pas être obtenues pour des compositions du même type obtenues classiquement et traitées subséquemment, dans des conditions classiques, par du charbon actif ou de l'eau oxygénée.

Ceci n'exclut aucunement d'ailleurs, qu'après les étapes d'estérification puis de purification, notamment par évaporation, le procédé conforme à l'invention puisse comprendre au moins une étape de traitement de la composition résultante par du charbon actif ou de l'eau oxygénée.

Le traitement par du charbon actif se fait par exemple par mise en contact de la composition avec 1 - 3 % en poids de charbon actif (noir en poudre) à une température voisine de 100 °C, puis par agitation à cette température pendant plusieurs dizaines de minutes, par exemple pendant environ une heure. A la fin du traitement, le charbon actif est séparé par filtration.

Un traitement classique de décoloration par eau oxygéné consiste par exemple à introduire dans la composition à décolorer, sur une période allant par exemple de 30 à 60 minutes, de 0,5 à 2 % d'eau oxygénée à 100 % à une température comprise entre 90 °C et 100 °C, puis agitation de la composition pendant une à deux heures à cette température.

Lorsque l'on souhaite associer ces deux types de traitement de décoloration, le traitement à l'eau oxygénée précède de préférence celui par le charbon actif. Ce dernier permet en effet de détruire les peroxydes éventuellement présents.

Il a été observé que la mise en oeuvre d'acide hypophosphoreux lors de l'étape d'estérification conformément à l'invention permettait d'obtenir, après évaporation puis simple traitement par charbon actif dans les conditions indiquées ci-dessus, une composition de diester(s) de dianhydrohexitol présentant un indice de jaune YI au plus égal à 25, voire au plus égal à 20. Ceci est d'autant plus remarquable que selon les nombreux travaux de recherche et d'analyse effectués par la Demanderesse, des valeurs si faibles ne pourraient être obtenues, en l'absence de mise en oeuvre d'acide hypophosphoreux, que par un traitement associant du charbon actif avec soit de l'eau oxygénée soit avec une quantité importante de n-hexane.

La présente invention a donc pour objet une composition de diester(s) de dianhydrohexitol, non traitée par l'eau oxygénée ou le n-hexane, obtenue par le procédé selon l'invention, caractérisée en ce qu'elle présente un indice de jaune YI au plus égal à 25, en particulier au plus égal à 20.

Ladite composition ainsi caractérisée peut avantageusement présenter une richesse en diester(s) au moins égale à 95 %, de préférence au moins égale à 98 %.

Il a également été constaté que la mise en oeuvre d'acide hypophosphoreux lors de l'étape d'estérification permettait notamment d'obtenir, après évaporation et simple traitement par de l'eau oxygénée dans les conditions indiquées ci-dessus, une composition de diester(s) de dianhydrohexitol présentant un indice de jaune YI au plus égal à 15, voire au plus égal à 10.

La Demanderesse considère qu'une telle composition n'a jamais été obtenue dans l'art antérieur ou n'aurait pu être obtenue sauf, peut-être, à imaginer de mettre en oeuvre des quantités d'eau oxygénée largement supérieures à celles pouvant être considérées comme « classiques », i.e. des quantités largement supérieures à 1 - 2 %.

La présente invention a également pour objet une composition de diester(s) de dianhydrohexitol, traitée par l'eau oxygénée, obtenue par le procédé selon l'invention, et caractérisée en ce qu'elle présente un indice de jaune YI au plus égal à 15, en particulier au plus égal à 10. Cet indice peut même être au plus égal à 7.

Ladite composition ainsi caractérisée présente de préférence une richesse en diester(s) au moins égale à 95 %, de préférence au moins égale à 98 %.

Selon une autre variante, il est en outre prévu qu'après les étapes d'estérification puis de purification, notamment par évaporation, le procédé conforme à l'invention comprenne, dans un ordre quelconque, au moins une étape de traitement par charbon actif et au moins une étape de traitement par eau oxygénée.

Selon un mode de réalisation préféré, l'étape de traitement par charbon actif est effectuée après l'étape de traitement par eau oxygénée.

De manière tout à fait remarquable, il a été observé que la mise en oeuvre d'acide hypophosphoreux lors de l'étape d'estérification conformément à l'invention permettait notamment d'obtenir, après évaporation puis traitement par charbon actif et/ou eau oxygénée, une composition de diester(s) de dianhydrohexitol présentant un indice de jaune YI au plus égal à 9, voire au plus égal à 7.

Ce résultat est d'autant plus surprenant que les nombreux travaux de recherche et d'analyse menés par la Demanderesse ont démontré que des valeurs aussi basses d'indice de jaune n'avaient en aucun cas été obtenues dans l'art antérieur, y compris selon l'EXEMPLE 5 de la demande de brevet WO 01/83488 précitée mené par ailleurs en présence de n-hexane, et n'auraient pu de toute façon être obtenues et ce, même en imaginant associer entre eux traitement par charbon actif (y compris dès l'étape d'estérification comme prévu dans l'EXEMPLE 5 précité) et traitement par eau oxygénée, dans des conditions que l'homme de l'art auraient jugées comme raisonnables.

La présente invention a par conséquent également pour objet une composition de diester(s) de dianhydrohexitol, obtenue par le procédé selon l'invention, caractérisée en ce qu'elle présente un indice de jaune YI au plus égal à 9, en particulier au plus égal à 7. Cette valeur peut en outre être au plus égale à 6, voire au plus égale à 5.

Ladite composition ainsi caractérisée peut avantageusement présenter une richesse en diester(s) au moins égale à 95 %, de préférence au moins égale à 98 %.

Ladite composition est en outre de préférence caractérisée par le fait qu'elle est exempte de traces de n-hexane.

En suite de quoi, on dispose désormais d'un moyen industriel particulièrement simple, économique, performant et sans danger, de préparation de compositions présentant des richesses élevées en diester(s) de dianhydrohexitol et qui sont moins colorées que celles de l'état de la technique.

Les compositions selon l'invention ou obtenues après purification, notamment par évaporation, selon l'une quelconque des variantes du procédé selon l'invention présentent avantageusement une richesse en diester(s) au moins égale à 95 %, de préférence au moins égale à 98 %.

Selon une autre variante, ces compositions sont exemptes de traces de xylène, de diéthyléther et de n-hexane, i.e de traces des solvants organiques utilisés dans les exemples des demandes internationales WO 99/45060 et WO 01/83488 précitées.

Ces compositions peuvent être mises en oeuvre, entre autres, dans l'une quelconque des applications décrites ou envisagées dans les documents précités de l'état de la technique.

Elles peuvent notamment être utilisées comme additifs, en particulier comme agents plastifiants, solvants, lubrifiants ou tensioactifs, dans la préparation de compositions de matières plastiques, de compositions bitumineuses ou résiniques, de compositions cellulosiques, de compositions destinées aux industries de la chimie, de la pharmacie, de la cosmétologie, de l'alimentation, humaine ou animale.

Les compositions selon l'invention peuvent consister, entre autres, en compositions nouvelles de :
- di-acétate d'isosorbide, d'isommanide et/ou d'isoidide,
- di n-octanoate d'isosorbide, d'isommanide et/ou d'isoidide,
- di-éthyl-2-hexanoate d'isosorbide, d'isomannide et/ou d'isoidide.

Concernant la préparation spécifique de diesters d'isoidide, préparation qui n'a jamais été exemplifiée jusqu'alors, il a été constaté en outre que le procédé conforme à l'invention permettait d'obtenir de tels produits dans des conditions encore plus favorables que dans le cas des diesters d'isosorbide et notamment :
- pour des durées réactionnelles d'estérification significativement plus courtes, par exemple en 3,5 heures au lieu de 5 heures, et
- aboutissant à des produits, par exemple des bruts réactionnels évaporés, éventuellement traités sur charbon actif, de coloration encore améliorée.

La Société Demanderesse considère d'ailleurs qu'une composition présentant une richesse en diester(s) d'isoidide au moins égale à 95 %, obtenue par le procédé selon l'invention, constitue un produit nouveau en tant que tel, indépendamment de toute autre caractéristique.

Une telle composition présente de préférence un indice de jaune YI au plus égal à 50.

Il peut s'agir en particulier d'une composition présentant une richesse, par exemple en di-n-octanoate et/ou di-éthyl-2-hexanoate d'isoidide, au moins égale à 98 % et un indice de jaune YI au plus égal à 25.

Une telle composition peut également être caractérisée en ce qu'elle est exempte de traces de xylène, diéthyléther et n-hexane.

De telles compositions se sont révélées être des compositions plastifiantes de matières plastiques ou bitumineuses aussi performantes, voire plus performantes pour certains aspects, que les compositions correspondantes à base de diester(s) d'isosorbide.

Compte-tenu de tout ce qui précède, la Société Demanderesse considère par ailleurs qu'un objet de la présente invention consiste globalement en l'utilisation d'acide hypophosphoreux lors de l'estérification d'un dianhydrohexitol, on présence d'un catalyseur acide.

La présente invention va être décrite de façon encore plus détaillée à l'aide des exemples qui suivent

### EXEMPLE 1

On réalise un essai conforme à l'invention (α ESSAI 1 ») selon le protocole opératoire général suivant.

Dans un réacteur en verre de 1 litre muni d'une double-enveloppe alimentée par un bain thermostaté à circulation d'huile, d'une påle d'agitation de type hélice, d'un thermomètre, d'une tête de distillation associée à un réfrigérant et à un collecteur de distillation, on introduit 146 g d'isosorbide (1 mole) et 432 g d'acide n-octanoïque (3 moles).

Le système d'agitation est mis en fonctionnement à 400 tr/mn, ainsi que le bain thermostaté avec une consigne de 100°C. Quand la température du milieu réactionnel atteint 60°C, on ajoute 2,92 g d' acide p-toluène sulfonique (APTS) monohydrate (ce qui correspond à 1,8 % en poids sec par rapport à l'isosorbide sec) et 0,90 g d'acide hypophosphoreux à 50 %, soit 0,3 % en poids de matière sèche par rapport à l'isosorbide sec et selon un ratio de 0,15/1 environ par rapport à l'APTS.

La consigne du bain thermostaté est ensuite fixée à 150°C et l'agitation à 650 tr/mn. L'ensemble du montage est alors relié à une pompe à vide munie d'un vacuuomètre dont on fixe la consigne à 100 mbars.

Lorsque la température du milieu réactionnel atteint 115°C environ, l'eau issue de la réaction d'estérification est distillée et recueillie dans le collecteur. Après 2 heures de réaction, la quantité d'eau distillée correspond à environ 85 % de la quantité d'eau théorique pour une réaction totale. Le vide est alors progressivement abaissé sur 3 heures supplémentaires jusqu'à 25 mbars, pendant que la température du milieu réactionnel atteint naturellement 140°C. Après 5 heures de réaction, l'eau distillée atteint 97 % de la théorie.

Le milieu réactionnel est ensuite refroidi jusqu'à 100°C environ, et on neutralise les acidités fortes de l'APTS et de l'acide hypophosphoreux par l'ajout de 1,8 g de soude à 50 %. Après précipitation des sels formés, le milieu réactionnel est filtré et se présente alors sous la forme d'un liquide limpide jaune. L'indice de jaune YI de ce brut réactionnel neutralisé / filtré, mesuré comme décrit précédemment, donne une valeur de 50,7.

Après remise dans le réacteur, on distille sous vide l'acide octanoïque qui n'a pas réagi (5 mbars ; température de vapeur : 115°C). La température du bouilleur évolue de 130 à 200°C environ pendant cette évaporation. La composition ainsi purifiée présente une richesse en di n-octanoate d'isosorbide de 98,5 %.

Cette richesse est mesurée par chromatographie phase gazeuse sur un appareillage de type Varian 3400 avec détection FID et injecteur split/splitless type 1077. La colonne utilisée est une DB1 de marque J & W Scientific de longueur 30 mètres, de diamètre interne 0,32 mm, d'épaisseur de film 0.25 µm. Les conditions de température sont : injecteur et détecteur : 300°C ; colonne : programmation de 100°C jusque 320°C à raison de 7°C/mn, maintien 10 mn à 320°C. L'injection se fait en split à 80 ml/mn, la pression en tête de colonne étant de 14 psi et le gaz vecteur utilisé étant de l'hélium.

La richesse est donnée, après normalisation interne, par la somme des proportions relatives des aires des composés dont le temps de rétention relatif est compris entre 1,52 et 1,72.

En outre, et de manière remarquable, la coloration de la composition ayant subi une évaporation n'est pas dégradée par rapport à celle du brut réactionnel neutralisé / filtré obtenu avant évaporation. En effet, ladite composition obtenue donc conformément à l'invention, présente un indice YI de 49,8.

Dans le cadre d'un essai témoin mené en l'absence de toute mise en oeuvre d'acide hypophosphoreux lors de la réaction d'estérification (« ESSAI T1 »), on a obtenu, dans les mêmes conditions générales :
- non seulement un brut réactionnel neutralisé / filtré de coloration brune (indice YI de 106,1),
- mais encore, après évaporation, une composition présentant une coloration encore plus prononcée (indice YI de 149,4).

Cette composition présentait en outre une richesse en di n-octanoate d'isosorbide inférieure à 98,5 %.

### EXEMPLE 2

D'autres essais (ci-après ESSAIS 2 à 7), également selon l'invention, ont été menés conformément au protocole opératoire général décrit pour l'ESSAI 1, si ce n'est que les modifications suivantes ont été apportées :
- ESSAI 2 : la réaction d'estérification a été menée pendant une durée de 6 heures au lieu de 5 heures,
- ESSAI 3 : les 1,8 % en poids sec d'APTS ont été remplacés par 1,1 % d'acide méthanesulfonique,
- ESSAI 4 : les 1,8 % en poids sec d'APTS ont été remplacés par 11 % de résine macroporeuse de type « Amberlyst 15 (Dry) »,
- ESSAI 5 : les 1,8 % en poids sec d'APTS ont été remplacés par 1,9 % en poids sec d'acide phosphotungstique, la réaction d'estérification étant menée sous balayage d'azote et non pas sous vide,
- ESSAI 6 : l'acide n-octanoïque a été remplacé (mole/mole) par de l'acide 2-éthylhexanoïque, la réaction d'estérification étant menée par ailleurs pendant une durée de 7,5 heures, à une température de 160 - 175 °C et sous un vide de 100 mbars,
- ESSAI 7 : l'isosorbide a été remplacé par de l'isoidide, la réaction d'estérification ayant pu être menée par ailleurs en 3,5 heures au lieu de 5 heures.

Le tableau ci-dessous reprend, pour chacun des ESSAIS 2 à 7 conformes à l'invention (mise en oeuvre de H₃PO₂ lors de l'estérification), l'indice de jaune obtenu pour le brut réactionnel neutralisé/filtré (ci-après « YI REAC »), l'indice de jaune obtenu subséquemment après évaporation (ci-après « YI EVAP ») et la richesse en diester obtenue pour la composition résultant de l'évaporation (ci-après « % RICH »), étant entendu que cette richesse concerne :
- le di n-octanoate d'isosorbide pour les ESSAIS 2 à 5,
- le di n-octanoate d'isoidide pour l'ESSAI 7, ladite richesse étant mesurée comme décrit précédemment pour le di- n octonoate d'isosorbide, et,
- le di 2-éthylhexanoate d'isosorbide pour l'ESSAI 6, ladite richesse étant mesurée comme décrit précédemment si ce n'est qu'après normalisation interne, on tient compte de la somme des proportions relatives des aires des composés dont le temps de rétention relatif est, cette fois, compris entre 1,44 et 1,55.

| ESSAI | YI REAC | YI EVAP | % RICH |
|---|---|---|---|
| 2 | 50,0 | 32,5 | 98 |
| 3 | 75,0 | 42,3 | 99 |
| 4 | 56,6 | 38,7 | 95 |
| 5 | 33,9 | 25,4 | 97 |
| 6 | 26,5 | 17, 6 | 99 |
| 7 | 71,5 | 40,6 | 99 |

Ces ESSAIS 2 à 7 confirment que les compositions conformes à l'invention peuvent présenter simultanément, après évaporation du brut réactionnel:
- non seulement une richesse élevée en diester(s) de dianhydrohexitol,
- mais aussi une coloration non dégradée, généralement améliorée, par rapport à celle du brut réactionnel.

De manière remarquable, cette coloration peut être caractérisée par un indice de jaune YI non seulement au plus égal à 50 mais également au plus égal à 45, voire 40, voire même 35.

Ces ESSAIS 2 à 7 montrent par ailleurs que dans le cadre de l'invention, on peut avantageusement remplacer une résine macroporeuse par toute une gamme d'autres catalyseurs acides tels que l'APTS, l'acide méthanesulfonique ou l'acide phosphotungstique.

En termes uniquement de richesse, ces catalyseurs acides se révèlent ici plus performants que la résine macroporeuse qui, dans le cas présent, permet cependant d'atteindre une richesse en diester de 95 %.

La Société Demanderesse a d'ailleurs observé, lors de travaux d'analyses approfondis, que dans le cas particulier d'une résine macroporeuse comme catalyseur, la majorité des impuretés présentes consistaient en esters, en particulier tétraesters, de sorbitan, ce qui laisse sous-entendre qu'en présence de ce type de catalyseur, le dianhydrohexitol subit une dégradation par ouverture de cycle lors de l'estérification.

Comme déjà souligné, le procédé selon l'invention présente également l'avantage insoupçonné de permettre la préparation efficace de nouvelles compositions non seulement de diester(s) d'isosorbide, par exemple de di n-octanoate d'isosorbide mais aussi de di-éthyl-2-hexanoate d'isosorbide, mais également de diester(s) d'isoidide, par exemple de di n-octanoate d'isoidide.

De manière spectactulaire, on observe que la mise en oeuvre d'acide hypophosphoreux lors de l'étape d'estérification de l'isoidide, laquelle étape peut d'ailleurs se dérouler en des durées réactionnelles remarquablement courtes (< 4 heures), génère un brut réactionnel dont la coloration (indice YI de 71,5) peut ensuite être diminuée de plus de 30 unités (indice YI de 40,6) lors de l'étape de purification par évaporation.

### EXEMPLE 3

Dans le cadre de cet exemple, on réalise des essais non conformes à l'invention (« ESSAIS T2 à T5) de la même manière que pour l'ESSAI T1 selon l'EXEMPLE 1, si ce n'est que les modifications suivantes ont été apportées :
- ESSAI T2 : IDEM ESSAI T1 sauf que l'estérification (en absence donc d'acide hypophosphoreux) a été menée en présence a) de résine « Amberlyst 15 (Dry)» (en remplacement de l'ATPS) mais aussi b) de charbon actif, conformément à l'EXEMPLE 5 de la demande WO 01/83488 précitée,

- ESSAI T3 : IDEM ESSAI T2 sauf qu'après évaporation du brut réactionnel et distillation concomitante de l'acide n-octanoïque en excès, la composition résultante a été soumise à un traitement supplémentaire de décoloration sur charbon actif,
- ESSAI T4 : IDEM ESSAI T3 sauf qu'après évaporation du brut réactionnel, la composition résultante a été soumise à un traitement supplémentaire par solvant (n-hexane) et charbon actif, conformément à l'EXEMPLE 5 du brevet WO 01/83488,
- ESSAI T5 : IDEM ESSAI T1 sauf que l'acide hypophosphoreux (H₃PO₂) a été remplacé (poids/poids) par de l'acide phosphorique (H₃PO₄).

Le tableau ci-dessous reprend, pour chacun des ESSAIS T2 à T5, non conformes à l'invention (absence de H₃PO₂ lors de l'estérification), les valeurs obtenues pour les critères prédéfinis « YI REAC », « YI EVAP » et « % RICH ».

| ESSAI | YI REAC | YI EVAP | % RICH |
|---|---|---|---|
| T2 | 22,5 | 52,7 | 94 |
| T3 | 22,5 | 30,9 | 94 |
| T4 | 22,5 | 28,2 | 94 |
| T5 | 132,0 | 180,1 | 97 |

Les résultats des ESSAIS T2 à T4 montrent que si la présence de charbon actif pendant la réaction d'estérification permet d'obtenir un brut réactionnel de coloration acceptable (indice YI de 22,5), il n'en demeure pas moins que :
- après évaporation (ESSAI T2), cette coloration se dégrade très significativement (indice YI augmenté de 30 unités environ) pour atteindre une valeur > 50 (52,7),
- un traitement subséquent par du charbon actif ne permet pas d'obtenir un indice YI au plus égal à 30 (YI de 30,9),
- un traitement subséquent par du n-hexane et du charbon actif ne permet pas d'obtenir un indice YI au plus égal à 25 (YI de 28,2).

Pour ces ESSAIS T2 à T4, la richesse en di-n-octanoate d'isosorbide est inférieure à 95 % en raison de la présence de teneurs importantes, de tétraesters de sorbitan, apparemment liée à l'usage de résine macroporeuse comme catalyseur acide d'estérification.

Les résultats de l'ESSAI T5 montrent par ailleurs que l'on ne peut remplacer l'acide hypophosphoreux par de l'acide phosphorique.

Ce dernier, non seulement ne permet pas d'obtenir un brut réactionnel dont l'indice YI est au plus égal à 50, la valeur trouvée (132,0) étant très significativement supérieure à cette limite, mais encore cette valeur élevée d'indice YI est très fortement augmentée par l'étape d'évaporation.

En outre, la richesse en di-n-octanoate d'isosorbide obtenue après évaporation pour cet ESSAI T5 n'était que de 97 %.

### EXEMPLE 4

Des essais conformes à l'invention (« ESSAIS 8 à 11 » ont été menés selon le protocole opératoire général décrit pour l'ESSAI 1 ou l'ESSAI 2, si ce n'est qu'après l'étape de distillation de l'acide octanoïque, la composition de diester résultante, purifiée par évaporation, a été soumise respectivement à chacun des traitements suivants :
- ESSAI 8 : ESSAI 1 + traitement par 2 % en poids (sec/sec) de charbon actif,
- ESSAI 9 : ESSAI 1 + traitement par 1 % en poids d'eau oxygénée à 100 %,
- ESSAI 10 : ESSAI 2 + traitement par 1 % en poids d'eau oxygénée à 100%
- ESSAI 11 : IDEM ESSAI 10 + traitement subséquent par 2 % en poids (sec/sec) de charbon actif.

L'ensemble des compositions conformes à l'invention ainsi préparées selon les EXEMPLES 8 à 11 ont montré une richesse élevée en di-n-octanoate d'isosorbide, de l'ordre de 99 %.

Le tableau ci-dessous, reprend, pour chacun desdits ESSAIS, la valeur d'indice de jaune YI obtenue pour la composition de diester résultante.

| | ESSAI 8 | ESSAI 9 | ESSAI 10 | ESSAI 11 |
|---|---|---|---|---|
| YI | 18,7 | 6,8 | 9,3 | 5,0 |

Il est tout à fait remarquable d'observer que, grâce au procédé selon l'invention, il est possible d'obtenir des compositions de diester(s) de dianhydrohexitol, n'ayant subi aucun traitement par un solvant organique, présentant :
- un indice de jaune au plus égal à 25, voire 20, en absence de tout traitement par eau oxygénée (cf. ESSAI 8), ou
- un indice de jaune au plus égal à 15, voire 10 (cf. ESSAI 10), voire même 7 (cf. ESSAI 9) par simple traitement par eau oxygénée, en l'absence de tout traitement par charbon actif,
- un indice de jaune au plus égal à 9, voire 7, voire même 6 ou 5, par traitement avec des proportions « raisonnables » d'eau oxygénée puis de charbon actif (cf. ESSAI 11).

Ces résultats sont d'autant plus surprenants que des essais témoins, menés conformément à l'ESSAI T1 (absence d'acide hypophosphoreux lors de l'estérification) mais envisageant, après l'étape d'évaporation, des traitements avec soit 2 % d'eau oxygénée à 100 %, soit 1 % d'eau oxygénée puis 2 % de charbon actif, n'ont pas permis de préparer des compositions de di n-octanoate d'isosorbide présentant un indice de jaune YI au plus égal à 20.

## Revendications

1. Procédé de préparation d'une composition de diester(s) de dianhydrohexitol, **caractérisé en ce qu'**il comprend une étape au cours de laquelle on soumet une composition de dianhydrohexitol à une estérification par un acide carboxylique en présence d'un catalyseur acide et d'acide hypophosphoreux.

2. Procédé selon la revendication 1, **caractérisé en ce que** l'acide hypophosphoreux est introduit en une quantité comprise entre 0,05 et 2 %, de préférence comprise entre 0,1 et 1 %, exprimée en poids sec par rapport au poids sec de dianhydrohexitol (s) mis en oeuvre.

3. Procédé selon la revendication 1 ou la revendication 2, **caractérisé en ce que** l'acide hypophosphoreux est introduit selon un ratio acide hypophosphoreux/catalyseur acide inférieur à 1/1, de préférence compris entre 0,01/1 et 0,9/1.

4. Procédé selon l'une des revendications 1 à 3, **caractérisé en ce qu'**il comprend une étape subséquente d' évaporation du milieu issu, directement ou non, de l'étape d' estérification,

5. Procédé selon la revendication 4, **caractérisé en ce qu'**il comprend au moins une étape subséquente de traitement de la composition résultante par du charbon actif et/ou de l'eau oxygénée.

6. Composition de diester(s) de dianhydrohexitol obtenue par le procédé selon l'une des revendications 4 à 5, et en ce richesse en diester(s) au moins égale à 95 % et un indice de jaune YI au plus égal à 50

## Claims

1. A method for preparing a composition of dianhydrohexitol diester(s), **characterized in that** it comprises a step during which a dianhydrohexitol composition is subjected to an esterification by a carboxylic acid in the presence of an acid catalyst and hypophosphorous acid.

2. The method as claimed in claim 1, **characterized in that** the hypophosphorous acid is introduced in an amount between 0.05 and 2%, preferably between 0.1 and 1%, expressed by dry weight relative to the dry weight of dianhydrohexitol(s) used.

3. The method as claimed in claim 1 or claim 2, **characterized in that** the hypophosphorous acid is introduced in a hypophosphorous acid/acid catalyst ratio less than 1/1, preferably between 0.01/1 and 0.9/1.

4. The method as claimed in one of claims 1 to 3, **characterized in that** it comprises a subsequent step of evaporation of the medium derived, directly or indirectly from the esterification step.

5. The method as claimed in claim 4, **characterized in that** it comprises at least one subsequent step of treating the resultant composition with activated carbon and/or hydrogen peroxide.

6. A composition of dianhydrohexitol obtained by the method as claimed in claim 4 or claim 5 and **characterized in that** it has a content of diester(s) at least equal to 95% and a yellow index YI at most equal to 50.

## Patentansprüche

1. Verfahren zur Herstellung einer Dianhydrohexitoldiesterzusammensetzung, **dadurch gekennzeichnet, dass** es einen Schritt umfasst, währenddessen man eine Dianhydrohexitolzusammensetzung einer Veresterung mit einer Carbonsäure in Gegenwart eines Säurekatalysators und von Phosphinsäure unterzieht.

2. Verfahren gemäß Anspruch 1, **dadurch gekennzeichnet, dass** die Phosphinsäure in einer Menge zwischen 0,05 und 2%, bevorzugt zwischen 0,1 und 1%, ausgedrückt als Trockengewicht bezogen auf das Trockengewicht des/der eingesetzten Dianhydrohexitols/Dianhydrohexitole, eingesetzt wird.

3. Verfahren gemäß Anspruch 1 oder Anspruch 2, **dadurch gekennzeichnet, dass** die Phosphinsäure in einem Verhältnis von Phosphinsäure/Säurekatalysator von kleiner als 1/1, bevorzugt zwischen 0,01 /1 und 0,9/1 eingesetzt wird.

4. Verfahren gemäß einem der Ansprüche 1 bis 3, **dadurch gekennzeichnet, dass** es einen nachfolgenden Schritt der Verdampfung des Mediums, das direkt oder nicht aus dem Schritt der Veresterung stammt, umfasst.

5. Verfahren gemäß Anspruch 4, **dadurch gekennzeichnet, dass** es mindestens einen nachfolgenden Schritt der Behandlung der resultierenden Zusammensetzung mit Aktivkohle und/oder Wasserstoffperoxid umfasst.

6. Dianhydrohexitoldiesterzusammensetzung, erhältlich durch das Verfahren gemäß einem der Ansprüche 4 bis 5, und wobei sie einen Diestergehalt von mindestens gleich 95% und einen Gelbwert YI von höchstens gleich 50 aufweist.
